# EUROPEAN PATENT APPLICATION

(11) **EP 4 702 974 A1**
(43) Date of publication of application: **04.03.2026**
(21) Application number: 23887181.8
(22) Date of filing: 25.05.2023
(51) Int. Cl.: A61K 9/08, A61K 31/662, A61K 47/10, A61K 47/02, A61K 47/18, A61K 47/26, A61P 27/02, A61P 29/00

(54) **OPHTHALMIC COMPOSITION CONTAINING VVN001**

(30) Priority: 27.04.2023 CN 202310476403
(71) Applicant: Vivavision Biotech Ltd., Zhejiang 325088 (CN)
(72) Inventor: XIA, Erning, Shanghai 201203 (CN); HAN, Qiao, Shanghai 201203 (CN); SHEN, Wang, Shanghai 201203 (CN); LU, Yangqingqin, Shanghai 201203 (CN); GAO, Hongyan, Shanghai 201203 (CN)
(74) Representative: Ostertag & Partner Patentanwälte mbB
(86) International application number: PCT/CN2023/096273
(87) International publication number: WO 2024/221527

(57) **Abstract**

The present application provides a VVN001-containing ophthalmic composition comprising an active material selected from a sodium salt represented by formula I and/or a free acid thereof, and ophthalmic excipients, to reduce the ocular inflammatory response by inhibiting a T cell-mediated inflammatory response through the active material. The concentration of the buffering agent in current invention is very low and the final buffer capacity of this ophthalmic composition is very close to the buffering capacity of the tear, which is tear-friendly and comfortable, and to increase the average residence time of the active material of the present application, as well as to increase the bioavailability of the active material represented by formula I according to the present application.

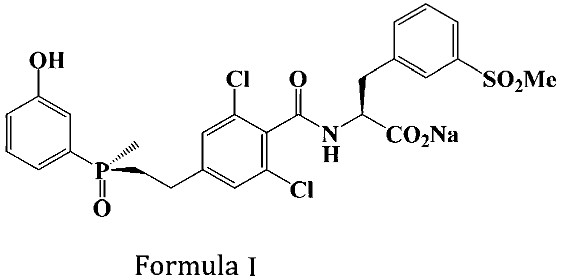

## Description

This application claims the benefit of priority to Chinese Patent Application No. 202310476403.2, filed before the CNIPA on April 27, 2023, entitled "A VVN001-CONTAINING OPHTHALMIC COMPOSITION", which is incorporated herein by reference in its entirety.

### TECHNICAL FIELD

The present application relates to the field of pharmaceutical technology, in particular to a VVN001-containing ophthalmic composition.

### BACKGROUND

Dry eye disease (DED), also known as dry keratoconjunctivitis sicca, is a complex disorder of ocular surface characterized by symptoms of visual disturbances, eye discomfort, and ocular dryness due to incompletion or instability, and uneven thickness of tear film. There is evidence indicating that T cells play a role to a certain extent in mediating inflammation. Heterodimeric receptors on T cells, called integrins, help T cells activate, adhere to the extracellular matrix, migrate, proliferate, and differentiate following inflammatory signals. In turn, lymphocyte function-associated antigen-1 (LFA-1)/intercellular adhesion molecule-1 (ICAM-1) interaction leads to T-cell adhesion to endothelial cells and migration to more inflamed tissues, where antigen presentation and recognition occurs, thereby promoting the formation of immune synapses. These immune synapses promote the propagation of downstream signals leading to the release of inflammatory mediators, cytokines, chemokines, TNF-α, and IL-1, which further exacerbate and continue inflammation in ocular tissues.

Therefore, there is a need for an eyedropper containing a small-molecule integrin antagonist against the mechanism of LFA-1/ICAM-1 interaction to treat dry eye disease and/or other ocular surface diseases by blocking the binding of LFA-1 and ICAM-1 to inhibit T cell-mediated inflammatory responses, thus reducing ocular inflammatory responses.

### SUMMARY

The object of the present application is to provide a tear-friendly and comfortable ophthalmic composition that achieves optimal bioavailability of the active component, reduces ocular inflammatory response, and treats dry eye disease and/or other ocular surface diseases. Specific technical solutions are as follows.

A first aspect of the present application provides a VVN001-containing ophthalmic composition comprising an active material and ophthalmic excipients, wherein the active material is a sodium salt represented by formula I and/or a free acid thereof. A content of the active material in the ophthalmic composition is 0.01 w/v% to 10 w/v%, preferably 0.1 w/v% to 7.5 w/v%, more preferably 1.0 w/v% to 5.0 w/v%.

The ophthalmic excipient comprises pH buffering agents and a tonicity agent. A content of the pH buffering agent in the ophthalmic composition is 0.001 w/v% to 2.0 w/v%, preferably 0.005 w/v% to 1.0 w/v%, more preferably 0.005 w/v% to 0.5 w/v%.

The pH of the ophthalmic composition is 6.5 to 7.8, preferably 7.0 to 7.4. The osmolarity is 200 mOsmol/L to 400 mOsmol/L, preferably 250 mOsmol/L to 350 mOsmol/L, more preferably 280 mOsmol/L to 320 mOsmol/L.

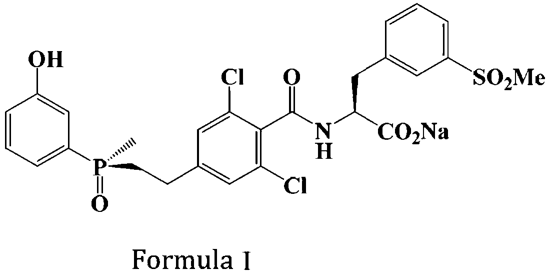

In one embodiment of the present application, the pH buffering agent is any one selected from the group consisting of boric acid, borate, citric acid, citrate, acetic acid-sodium acetate, trometamol hydrochloride, sodium bicarbonate, and phosphate.

The borate is at least one selected from the group consisting of sodium borate, potassium borate, and hydrates thereof.

The citrate is at least one selected from the group consisting of potassium citrate, sodium citrate, disodium hydrogen citrate, sodium dihydrogen citrate, dipotassium hydrogen citrate, potassium dihydrogen citrate, and hydrates thereof.

The phosphate is one or more selected from the group consisting of disodium hydrogen phosphate, sodium dihydrogen phosphate, dipotassium hydrogen phosphate, potassium dihydrogen phosphate, and hydrates thereof.

In one embodiment of the present application, the pH buffering agent is phosphate. A content of the phosphate in the ophthalmic aqueous composition is 0.001 w/v% to 2.0 w/v%, preferably 0.01 w/v% to 1.0 w/v%, more preferably 0.005 w/v% to 0.5 w/v%.

In one embodiment of the present application, the tonicity agent is selected from an inorganic tonicity agent and/or an organic tonicity agent.

The inorganic tonicity agent is one or more selected from the group consisting of sodium chloride, potassium chloride, calcium chloride, zinc chloride and magnesium chloride. A content of the inorganic tonicity agent in the ophthalmic composition in an aqueous solution is 0.01 w/v% to 1.5 w/v%, preferably 0.2 w/v% to 1 w/v%.

The organic tonicity agent is any one selected from the group consisting of erythritol, glucose, glycerol, propylene glycol, glycine, diglycine, alanine, taurine, tetrahydromethylpyrimidine carboxylic acid, mannitol, sorbitol, L-Carnitine, and trehalose. The content of the organic tonicity agent in the ophthalmic composition is 0.001 w/v% to 10 w/v%, preferably 0.01 w/v% to 5 w/v%.

In one embodiment of the present application, the ophthalmic excipient further comprises a surfactant. The surfactant is at least one selected from the group consisting of polyoxyethylene-polyoxypropylene-polyoxyethylene triblock copolymer, sodium dodecyl sulfate, polyethoxylated sorbitan fatty acid ester, polyoxyethylene alkyl ether, polyoxyethylene stearate, poloxamine, sorbitan fatty acid ester, polyethylene glycol, polyethoxylated fatty alcohol, polyoxyethylene 40 hydrogenated castor oil, docusate sodium, quaternary ammonium compounds, C₆-C₂₀ fatty acids, saccharide fatty acid esters, fatty acid glycerides, polysorbate, and tyloxapol. A content of the surfactant in the ophthalmic composition is 0.001 w/v% to 1 w/v%.

In one embodiment of the present application, the ophthalmic excipient further comprises a comfort agent. The comfort agent is at least one selected from the group consisting of polyol, cellulose derivative, dextran, polyethylene glycol, polysorbate, povidone, trehalose, hyaluronic acid, carbomer, sodium hyaluronate and sodium alginate.

The polyol is at least one selected from the group consisting of glycerol, propylene glycol, polyvinyl alcohol and mannitol.

The cellulose derivative is at least one selected from the group consisting of hydroxypropylmethyl cellulose-E4M, hydroxypropylmethyl cellulose-LV, hydroxyethyl cellulose, hydroxymethyl cellulose, methylcellulose, hemicellulose and ethylcellulose.

A content of the comfort agent in the ophthalmic composition is 0.001 w/v% to 2 w/v%.

In one embodiment of the present application, the ophthalmic excipient further comprises a chelating agent. The chelating agent is at least one selected from the group consisting of disodium ethylenediaminetetraacetate, alkali metal hexametaphosphate, nitrilotriacetic acid, ethylenediamine disuccinic acid, iminodisuccinic acid, methylglycine diacetic acid, L-glutamic acid N,N-diacetic acid, ethylenediamine-N,N'-diglutamic acid, ethylenediamine-N,N'-bismalonic acid, 3-hydroxy-2,2-iminodisuccinic acid, 2-hydroxyethyliminodiacetic acid, 2,6-pyridinedicarboxylic acid, diethylenetriaminepentaacetic acid, hydroxyethyldiaminetriacetic acid, 1,2-diaminocyclohexanetetraacetic acid, hydroxyethylaminodiacetic acid, polyphosphate, citric acid, citrate, tartaric acid, tartrate, and ethylenediaminetetraacetic acid. A content of the chelating agent in the ophthalmic composition is 0.001 w/v% to 1 w/v%.

The citrate is at least one selected from the group consisting of potassium citrate, sodium citrate, and hydrates thereof. The tartrate is at least one selected from the group consisting of sodium tartrate, potassium tartrate, potassium hydrogen tartrate, sodium hydrogen tartrate, and hydrates thereof.

In one embodiment of the present application, the ophthalmic excipient further comprises an antioxidant. The antioxidant is at least one selected from the group consisting of sodium thiosulfate, sodium metabisulfite, N-acetylcysteine, butylhydroxyanisole, and butylhydroxytoluene. A content of the antioxidant in the ophthalmic composition is 0.001 w/v% to 1.0 w/v%.

In one embodiment of the present application, the ophthalmic excipient further comprises a preservative. The preservative is at least one selected from the group consisting of benzalkonium chloride, sodium chlorite, polyquaternium-1, sorbic acid, ethylenediaminetetraacetic acid, boric acid, sodium borate, sodium bisulfate, sodium thiosulphate, ascorbate, urea peroxide, and benzalkonium bromide. A content of the preservative in the ophthalmic composition is 0.001 w/v% to 0.1 w/v%.

A second aspect of the present application provides the use of the ophthalmic composition described in any of the above embodiments in the preparation of a medicament for treatment of dry eye disease.

Beneficial effects of the present application are as follows.

The present application provides an ophthalmic composition against the mechanism of lymphocyte function-associated antigen-1 (LFA-1)/intercellular adhesion molecule-1 (ICAM-1) interaction to treat dry eye disease and/or other ocular surface diseases by blocking the binding of LFA-1 and ICAM-1 to inhibit T cell-mediated inflammatory responses, thus reducing ocular inflammatory responses, through a small-molecule integrin antagonist represented by formula I. The present application further selects a pH buffer at low concentration to match the pH buffer concentration to the buffering capacity in the tear, which is tear-friendly and comfortable, and to increase the average residence time of the active material of the present application, as well as to increase the bioavailability of the active material in the ophthalmic aqueous solution composition.

Of course, all of the advantages described above do have to be achieved at the same time when any of the products or methods of the present application is implemented.

### BRIEF DESCRIPTION OF THE DRAWINGS

The accompanying drawings illustrated herein are used to provide a further understanding of the present application and constitute part of the present application. The exemplary embodiments of the present application and the description thereof are used to explain the present application and do not constitute an undue limitation of the present application.

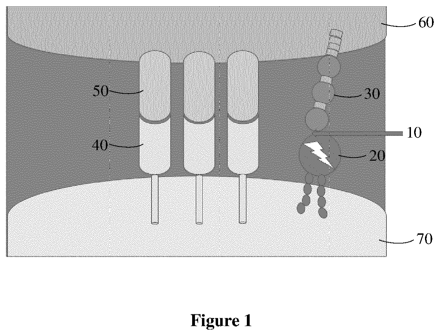

Figure 1 is a schematic diagram showing the mechanism of treatment of dry eye disease by an active material in the ophthalmic composition of the present application.

### DETAILED DESCRIPTION

The present application is further illustrated with the reference to the accompanying drawings and examples to make the objects, technical solution and advantages of the embodiments more apparent to those skilled in the art. Obviously, the described embodiments are only a portion of the embodiments of the present application and not all of them. On the basis of the embodiments in the present application, all other embodiments obtained by those skilled in the art fall within the scope of protection of the present application.

It should be noted that, as used herein, the terms "comprise" and any form thereof, such as "comprising" and "comprises", "have" and any form thereof, such as "having" and "has", "include" and any form thereof, such as "includes" and "including", or "contain" and any form thereof, such as "contains" and "containing" are inclusive or open-ended, so that a process, method or article comprising a set of elements includes not only those elements, but also other elements that are not explicitly listed, or elements inherent to such a process, method or article.

The first aspect of the present application provides a VVN001-containing ophthalmic composition comprising an active material and an ophthalmic excipient, wherein the active material is a sodium salt represented by formula I and/or a free acid thereof. The sodium salt represented by formula I is named chemically as sodium (S)-2-(2,6-dichloro-4-(2-((R)-(3-hydroxyphenyl)(methyl)phosphono)ethyl)benzamide)-3-(3-(met hylsulfonyl)phenyl)propionate, with a molecular formula of C₂₆H₂₅Cl₂NO₇PSNa and a molecular weight of 620.4, and the molecular weight of free acid thereof is 598.4.

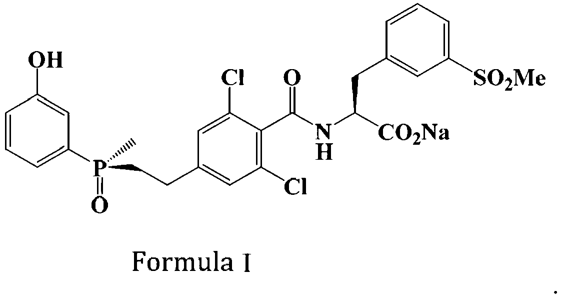

A content of the active material in the ophthalmic composition is 0.01 w/v% to 10 w/v%, preferably 0.1 w/v% to 7.5 w/v%, more preferably 1.0 w/v% to 5.0 w/v%. For example the content of the active material in the ophthalmic aqueous composition can be 0.01 w/v%, 0.025 w/v%, 0.05 w/v%, 0.10 w/v%, 0.25 w/v%, 0.50 w/v%, 1.0 w/v%, 2.0 w/v%, 3.0 w/v%, 4.0 w/v%, 5.0 w/v%, 6.0 w/v%, 7.0 w/v%, 8.0 w/v%, 9.0 w/v%, 10.0 w/v%, or a range between any two values therein. The unit of "w/v" in the application is g/ml.

The ophthalmic excipient comprises a pH buffer and a tonicity agent. A content of the pH buffer in the ophthalmic composition is 0.001 w/v% to 2.0 w/v%, preferably 0.005 w/v% to 1.0 w/v%, more preferably 0.005 w/v% to 0.5 w/v%. For example, the content of the pH buffer in the ophthalmic composition can be 0.001 w/v%, 0.005 w/v%, 0.01 w/v%, 0.025 w/v%, 0.05 w/v%, 0.10 w/v%, 0.25 w/v%, 0.50 w/v%, 1.0 w/v%, 1.5 w/v%, 2.0 w/v%, or a range between any two values therein.

The pH of the ophthalmic composition is 6.5 to 7.8, preferably 7.0 to 7.4. the osmolarity is 200 mOsmol/L to 400 mOsmol/L, preferably 250 mOsmol/L to 350 mOsmol/L, more preferably 280 mOsmol/L to 320 mOsmol/L. For example, the pH of the ophthalmic composition can be 6.5, 6.6, 6.7, 6.8, 6.9, 7.0, 7.1, 7.2, 7.3, 7.4, 7.6, 7.8 or a range between any two values therein. The osmolarity of the ophthalmic aqueous composition can be 200 mOsmol/L, 220 mOsmol/L, 250 mOsmol/L, 260 mOsmol/L, 270 mOsmol/L, 280 mOsmol/L, 290 mOsmol/L, 300 mOsmol/L, 320 mOsmol/L, 350 mOsmol/L, 380 mOsmol/L, 400 mOsmol/L, or a range between any two values therein.

The inventor found that, as shown in FIG. 1, lymphocyte function-associated antigen-1 (LFA-1) 20 interacts with intercellular adhesion molecule-1 (ICAM-1) 30, and that VVN001, as an active material 10 and being a small-molecule integrin antagonist, is able to block the binding of lymphocyte function-associated antigen-1 (LFA-1) 20 and intercellular adhesion molecule-1 (ICAM-1) 30 to inhibit the binding of T-cell receptor (TCR) 40 and major histocompatibility complex (MHC) 50 in the membrane of antigen-presenting cells (APCs) 60, inhibiting the formation of immune synapses between T-cells 70 and the membrane of antigen-presenting cells (APCs) 60, thereby reducing the ocular inflammatory response, thus achieving therapeutic effects for the treatment of dry eye disease and/or other ocular surface disorders. The inventor further found that by modulating the content of the active material in the range described above and modulating the pH buffer in the low concentration range described above, it is possible to match the pH buffer concentration with the buffering capacity in the tear (pH 7.0 to 7.4), which is tear-friendly and comfortable, and to increase the average residence time of the active material of the present application, as well as to increase the bioavailability of the active material described above. In the present application, VVN001 refers to the sodium salt represented by formula I and/or the free acid thereof.

In one embodiment of the present application, the pH buffer is any one selected from the group consisting of boric acid, borate, citric acid, citrate, acetic acid-sodium acetate, trometamol hydrochloride, sodium bicarbonate, and phosphate. The borate is at least one selected from the group consisting of sodium borate, potassium borate, and hydrates thereof. The citrate is at least one selected from the group consisting of potassium citrate, sodium citrate, disodium hydrogen citrate, sodium dihydrogen citrate, dipotassium hydrogen citrate, potassium dihydrogen citrate, and hydrates thereof. The phosphate is one or more selected from the group consisting of disodium hydrogen phosphate, sodium dihydrogen phosphate, dipotassium hydrogen phosphate, potassium dihydrogen phosphate, and hydrates thereof. The present application has no particular limitation on the method for preparing the pH buffer, and it can be formulated by methods known in the art, as long as the purpose of the present application can be realized. The citric acid and citrate can be used as a pH buffer or as a chelating agent, and the role of the citric acid and citrate in the ophthalmic composition can be determined according to the specific formulation. In present application, the above "the borate is at least one selected from the group consisting of sodium borate, potassium borate, and hydrates thereof" means that the borate can be at least one selected from the group consisting of sodium borate, potassium borate, sodium borate hydrates, and potassium borate hydrates, and other similar expressions should be understood by analogy. The present application does not specifically limit the hydrate of each material, which may be a conventional hydrate known in the art, as long as the purpose of the present application can be realized.

In one embodiment of the present application, the pH buffer is selected from the group consisting of phosphate. A content of the phosphate in the ophthalmic composition is 0.001 w/v% to 2.0 w/v%, preferably 0.005 w/v% to 1.0 w/v%, more preferably 0.005 w/v% to 0.5 w/v%. For example, the content of the phosphate in the ophthalmic composition can be 0.001 w/v%, 0.005 w/v%, 0.01 w/v%, 0.025 w/v%, 0.05 w/v%, 0.10 w/v%, 0.25 w/v%, 0.50 w/v%, 1.5 w/v%, 2.0 w/v%, or a range between any two values therein. The inventor found that by selecting a phosphate buffer system and modulating the content of phosphate within the above ranges, the phosphate concentration can be matched to the buffering capacity in the tear, minimizing the stimulus and/or discomfort experience from the higher ionic strength buffer system.

In one embodiment of the present application, the pH buffer is any one selected from the group consisting of boric acid, borate, citric acid, citrate, acetic acid-sodium acetate, trometamol hydrochloride, and sodium bicarbonate. The content of the pH buffer in the ophthalmic composition is 0.001 w/v% to 2.0 w/v%, preferably 0.005 w/v% to 1.0 w/v%, more preferably 0.005 w/v% ~0.5 w/v%.

In one embodiment of the present application, the tonicity agent is an inorganic tonicity agent and/or an organic tonicity agent.

The inorganic tonicity agent is one or more selected from the group consisting of sodium chloride, potassium chloride, calcium chloride, zinc chloride, and magnesium chloride. A content of the inorganic tonicity agent in the ophthalmic composition is 0.01 w/v% to 1.5 w/v%, preferably 0.2 w/v% to 1 w/v%. For example, the content of the inorganic tonicity agent in the ophthalmic composition may be 0.01 w/v%, 0.025 w/v%, 0.05 w/v%, 0.10 w/v%, 0.25 w/v%, 0.50 w/v%, 1.0 w/v%, 1.5 w/v% or a range between any two values therein.

The organic tonicity agent is any one selected from the group consisting of erythritol, glucose, glycerol, propylene glycol, L-Carnitine, and trehalose. A content of the organic tonicity agent in the ophthalmic composition is 0.001 w/v% to 10.0 w/v%, preferably 0.01 w/v% to 5.0 w/v%. For example, the content of the organic tonicity agent in the ophthalmic composition in aqueous solution may be 0.001 w/v%, 0.01 w/v%, 0.05 w/v%, 0.10 w/v%, 0.25 w/v%, 0.50 w/v%, 1.0 w/v%, 5.0 w/v%, 10.0 w/v%, or a range between any two values therein. The glycerol and propylene glycol may act as organic tonicity agents or as comfort agents, and the roles of the glycerol and propylene glycol in the ophthalmic composition may be determined according to the specific formulation. The inventors found that by selecting the above organic tonicity agents and/or inorganic tonicity agents and regulating the organic tonicity agents and/or inorganic tonicity agents in the above respective ranges, the osmolarity of the ophthalmic composition can be 280 mOsmol/L to 320 mOsmol/L, which is close to the osmolarity of the normal tear (270 mOsmol/L to 310 mOsmol/L), greatly reducing the discomfort of the patient when using the ophthalmic composition. Low content of the organic tonicity agent and/or the inorganic tonicity agent may lead to low osmolarity of the ophthalmic composition, while excessive content of tonicity agent may form a hypertonic solution. Both hypotonicity and hypertonicity can cause the crystalline lens to lose its desired optical parameters. Hypertonic solutions can also cause stinging, eye irritation, drying of the ocular surface, and the like. In one embodiment of the present application, the ophthalmic excipient further comprises a surfactant. The surfactant is at least one selected from the group consisting of polyoxyethylene-polyoxypropylene-polyoxyethylene triblock copolymer, sodium dodecyl sulfate, polyethoxylated sorbitan fatty acid ester, polyoxyethylene alkyl ether, polyoxyethylene stearate, poloxamine, sorbitan fatty acid ester, polyethylene glycol, polyethoxylated fatty alcohol, polyoxyethylene 40 hydrogenated castor oil, docusate sodium, quaternary ammonium compounds, C₆-C₂₀ fatty acids, saccharide fatty acid esters, fatty acid glycerides, polysorbate, and tyloxapol. A content of the surfactant in the ophthalmic composition is 0.001 w/v% to 1.0 w/v%. For example, the content of the surfactant in the ophthalmic composition may be 0.001 w/v%, 0.01 w/v%, 0.05 w/v%, 0.10 w/v%, 0.25 w/v%, 0.50 w/v%, 1.0 w/v%, or a range between any two values therein.

In the present application, the polyoxyethylene-polyoxypropylene-polyoxyethylene triblock copolymer is a specific class of block copolymers trademarked as Pluronic^{®}, which is also known as Poloxamer. a represents the number of aqueous group of hydrophilic polyethylene oxide (PEO) units in the block copolymer, and b represents the number of hydrophobic polypropylene oxide (PPO) units in the block copolymer. The hydrophilic-lipophilic balance HLB (equal to a/b) of the polyoxyethylene-polyoxypropylene-polyoxyethylene triblock copolymer increases with the increasing of a/b, resulting the copolymer being more hydrophilic. In the present application, the polyoxyethylene-polyoxypropylene-polyoxyethylene triblock copolymer, i.e. Pluronic^{®}, has an HLB > 10.

In one embodiment of the present application, the ophthalmic excipient further comprises a comfort agent. The comfort agent is at least one selected from the group consisting of polyol, cellulose derivative, dextran, polyethylene glycol, polysorbate, povidone, trehalose, hyaluronic acid, carbomer, sodium hyaluronate and sodium alginate. The polyol is at least one selected from the group consisting of glycerol, propylene glycol, polyvinyl alcohol and mannitol. The cellulose derivative is at least one cderivative selected from the group consisting of hydroxypropylmethyl cellulose-E4M (HPMC-E4M), hydroxypropylmethyl cellulose-LV (HPMC-LV), hydroxyethyl cellulose, hydroxymethyl cellulose, methylcellulose, hemicellulose and ethylcellulose. A content of the comfort agent in the ophthalmic composition is 0.001 w/v% to 2 w/v%. For example, the content of the comfort agent in the ophthalmic composition may be 0.001 w/v%, 0.01 w/v%, 0.05 w/v%, 0.10 w/v%, 0.25 w/v%, 0.50 w/v%, 1.0 w/v%, 2.0 w/v%, 10.0 w/v%, or a range between any two values therein.

In the present application, by adding the comfort agent and regulating the content of the comfort agent within the above range, the comfort of patients when using the ophthalmic composition is further improved.

In one embodiment of the present application, the ophthalmic excipient further comprises a chelating agent. The chelating agent is at least one selected from the group consisting of disodium ethylenediaminetetraacetate (EDTA), alkali metal hexametaphosphate, nitrilotriacetic acid, ethylenediamine disuccinic acid, iminodisuccinic acid, methylglycine diacetic acid, L-glutamic acid N,N-diacetic acid, ethylenediamine-N,N'-diglutamic acid, ethylenediamine-N,N'-bismalonic acid, 3-hydroxy-2,2-iminodisuccinic acid, 2-hydroxyethyliminodiacetic acid, 2,6-pyridinedicarboxylic acid, diethylenetriaminepentaacetic acid, hydroxyethyldiaminetriacetic acid, 1,2-diaminocyclohexanetetraacetic acid, hydroxyethylaminodiacetic acid, polyphosphate, citric acid, citrate, tartaric acid, tartrate, and ethylenediaminetetraacetic acid. The citrate is at least one selected from the group consisting of potassium citrate, sodium citrate, and hydrates thereof. The tartrate is at least one selected from the group consisting of sodium tartrate, potassium tartrate, potassium hydrogen tartrate, sodium hydrogen tartrate, and hydrates thereof. A content of the chelating agent in the ophthalmic composition is 0.001 w/v% to 1.0 w/v%. For example, the content of the chelating agent in the ophthalmic composition in an aqueous solution can be 0.001 w/v%, 0.005 w/v%, 0.01 w/v%, 0.025 w/v%, 0.05 w/v%, 0.10 w/v%, 0.25 w/v%, 0.50 w/v%, 1.0 w/v%, or a range between any two values therein. The above "the tartrate is at least one selected from the group consisting of sodium tartrate, potassium tartrate, potassium hydrogen tartrate, sodium hydrogen tartrate, and hydrates thereof" means that the tartrate is at least one selected from the group consisting of sodium tartrate, potassium tartrate, potassium hydrogen tartrate, sodium hydrogen tartrate and hydrates of the above salts, and the hydrates of the above salts are known conventional hydrates of the corresponding salts.

In one embodiment of the present application, the ophthalmic excipient further comprises an antioxidant. The antioxidant is at least one selected from the group consisting of sodium thiosulfate, sodium metabisulfite, N-acetylcysteine, butylhydroxyanisole (BHA), and butylhydroxytoluene (BHT). A content of the antioxidant in the ophthalmic composition is 0.001 w/v% to 1.0 w/v%. For example, the content of the antioxidant in the ophthalmic composition can be 0.001 w/v%, 0.005 w/v%, 0.01 w/v%, 0.025 w/v%, 0.05 w/v%, 0.10 w/v%, 0.25 w/v%, 0.50 w/v%, 1.0 w/v%, or a range between any two values therein.

In one embodiment of the present application, the ophthalmic excipient further comprises a preservative. The preservative is at least one selected from the group consisting of benzalkonium chloride (BAK), sodium chlorite, polyquaternium-1, sorbic acid, ethylenediaminetetraacetic acid, boric acid, sodium borate, sodium bisulfate, sodium thiosulphate, ascorbate, urea peroxide, and benzalkonium bromide. A content of the preservative in the ophthalmic composition is 0.001 w/v% to 0.1 w/v%. For example, the content of the preservative in the ophthalmic composition can be 0.001 w/v%, 0.005 w/v%, 0.01 w/v%, 0.025 w/v%, 0.05 w/v%, 0.10 w/v%, or a range between any two values therein.

In one embodiment of the present application, the active material is selected from the group consisting of the sodium salt represented by formula I. The method for preparing the ophthalmic composition comprises the following steps:
- adding water for injection equivalent to 85% to 90% of the total volume of the ophthalmic composition into a container; slowly adding the following materials in sequence with constantly stirring: a pH buffer, a tonicity agent and additional ophthalmic excipients, and then stirring the mixture for not less than 10 min;
- to the solution obtained above, adding the sodium salt represented by formula I as the active material, stirring the mixture for not less than 20 min, and then adjusting the pH of the solution to 6.5 to 7.8 with 1 N NaOH or 1 N HCl;
- adding water for injection to bring the volume to 100% of the total volume of the ophthalmic composition, and continuing the stirring for not less than 15 min; and sterilizing the resultant solution to obtain the VVN001-containing ophthalmic composition.

In one embodiment of the present application, the active material is the free acid of the sodium salt represented by formula I. The method for preparing the VVN001-containing ophthalmic composition comprises the following steps:
- adding water for injection which accounts for 40% to 50% of the total volume of the ophthalmic composition into a container; adding sodium hydroxide with stirring and stirring the solution for not less than 15 min; then adding the free acid of the sodium salt represented by formula I to the above solution, and stirring the mixture until free acid was dissolved;
- adding water for injection again to bring the solution volume to 85% to 90% of the total volume of the ophthalmic composition; slowly adding the following materials in sequence: a pH buffer, a tonicity agent and an ophthalmic excipient, and then stirring for not less than 10 min;
- adjusting the pH of the solution obtained above to 6.5 to 7.8 with 1 N NaOH or 1 N HCl;
- adding water for injection for dilute to volume to bring the volume of the solution to 100% of the total volume of the ophthalmic composition; continuing the stirring for not less than 15 min after the completion of diluting to volume; and sterilizing the resultant solution to obtain the VVN001-containing ophthalmic composition.

The preparation method of the present application achieves sterility, desirable stability and extremely low total impurity content of the VVN001-containing ophthalmic composition.

In the present application, there is no particular limitation on the sterilization means in the preparation method, as long as the purpose of the present application can be achieved, for example, sterilization can be carried out by terminal heat sterilization, filtration sterilization, electron-beam sterilization, UV system and the like, and specifically, for example, the filtration sterilization can be carried out by using a 0.22 µm sterile filter or the heat sterilization can be carried out at 121°C for not less than 30 min.

In the present application, there is no particular limitation on the forms for bottling the VVN001-containing ophthalmic composition, as long as the purpose of the present application can be realized, for example, it can be a conventional single-dose bottle and a BFS (blow-fill-seal) single use bottle or a multi-dose bottle.

A second aspect of the present application provides the use of the VVN001-containing ophthalmic composition described in any of the above embodiments in the preparation of a medicament for treatment of dry eye disease. The inventor found that the active material in the VVN001-containing ophthalmic composition, which is a sodium salt represented by formula I and/or free acid thereof and a small-molecule integrin antagonist, can treat dry eye disease and/or other ocular surface diseases by blocking the binding of LFA-1 and ICAM-1 to inhibit T cell-mediated inflammatory responses, thus reducing ocular inflammatory responses.

In the present application, the ophthalmic composition can be used and dosed as one drop/eye once or twice daily.

In the present application, the VVN001-containing ophthalmic composition can be used topically on the eyes, ears and nose.

### Examples

### Example 1

### Preparation of VVN001-containing ophthalmic composition

Water for injection equivalent to 85% of the total volume of the ophthalmic composition was added to a stainless steel mixing vessel. It was continuously stirred and slowly added with the following ophthalmic excipients in sequence: sodium dihydrogen phosphate monohydrate, sodium chloride, and sodium thiosulfate pentahydrate, and then the solution was stirred for 20 min. The sodium salt represented by formula I was added to the solution obtained above and the resultant was stirred for 30 min. The pH of the solution was adjusted to 7.2 with 1 N NaOH or 1 N HCl. Water for injection was added for diluting to volume. The mixture was continuously stirred for 20 min. The ophthalmic composition was obtained by filtration sterilization through a 0.22 µm sterile filter.

The product parameters (including composition, assay, pH and osmolarity) of the ophthalmic composition obtained in Example 1 are shown in Table 1.

### Example 2

Example 2 was performed in steps as same as Example 1 except that the product parameters were adjusted to those shown in Table 2.

### Example 3

Example 3 was performed in steps as same as Example 1 except that the product parameters were adjusted to those shown in Table 3.

### Example 4

Example 4 was performed in steps as same as Example 1 except that the product parameters were adjusted to those shown in Table 4.

### Example 5

Example 5 was performed in steps as same as Example 1 except that the product parameters were adjusted to those shown in Table 5.

### Example 6

Example 6 was performed in steps as same as Example 1 except that the product parameters were adjusted to those shown in Table 6.

### Example 7

Example 7 was performed in steps as same as Example 1 except that the product parameters were adjusted to those shown in Table 7.

### Example 8

Example 8 was performed in steps as same as Example 1 except that the product parameters were adjusted to those shown in Table 8.

### Example 9

Example 9 was performed in steps as same as Example 1 except that the product parameters were adjusted to those shown in Table 9.

### Example 10

Example 10 was performed in steps as same as Example 1 except that the product parameters were adjusted to those shown in Table 10.

### Example 11

Example 11 was performed in steps as same as Example 1 except that the product parameters were adjusted to those shown in Table 11.

### Example 12

Example 12 was performed in steps as same as Example 1 except that the product parameters were adjusted to those shown in Table 12.

### Example 13

Example 13 was performed in steps as same as Example 1 except that the product parameters were adjusted to those shown in Table 13.

### Example 14

Example 14 was performed in steps as same as Example 1 except that the product parameters were adjusted to those shown in Table 14.

### Example 15

Example 15 was performed in steps as same as Example 1 except that the product parameters were adjusted to those shown in Table 15.

### Examples 16

Example 16 was performed in steps as same as Example 1 except that the product parameters were adjusted to those shown in Table 16.

### Examples 17

Water for injection which accounts for 40% of the total volume of the ophthalmic composition was added to a stainless steel mixing vessel. Sodium hydroxide was added with stirring, and the solution was stirred for 20 min. Then the active material which is the free acid of the sodium salt represented by formula I was added to the above solution, and the mixture was stirred until the active material was dissolved.

Water for injection was added again to bring the mixture volume to 85% of the total volume of the ophthalmic composition. The following materials were added slowly in sequence: sodium dihydrogen phosphate monohydrate, sodium chloride, sodium thiosulfate pentahydrate, and benzalkonium chloride. The resultant was stirred for 20 min and the pH thereof was adjusted to 7.2 with 1 N NaOH or 1 N HCl. Water for injection is added for diluting to volume. The solution was continuously stirred for 20 min after the completion of diluting to volume. The VVN001-containing ophthalmic composition was obtained by filtration sterilization through a 0.22 µm sterile filter. The product parameters (including composition, content, pH and osmolarity) of the ophthalmic composition obtained in Example 17 are shown in Table 17.

**Table 1**

| Composition | Content (w/v%) | FDA Maximum Potency per Unit Dose IID (w/v%) | Function |
|---|---|---|---|
| Sodium Salt Represented by Formula I | 5.0 | - | Active Material |
| Sodium Phosphate, monobasic, Monohydrate | 0.02 | 0.041-1.3 | pH Buffer |
| Sodium Chloride | 0.4 | 0.65-0.9 | Inorganic tonicity agent |
| Sodium Thiosulfate Pentahydrate | 0.4 | 0.2-0.31 | Antioxidant |
| Benzalkonium Chloride | 0.02 | 0.005-1.0 | Preservative |
| Water for Injection | Solvent | | |
| pH (after the adjustment by 1 N NaOH or 1N HCl) | 7.2 ± 0.2 | | |
| Osmolarity | 300 ± 20 mOsmol/L | | |

| | | | |
|---|---|---|---|
| Note: the "IID content standards" in Table 1 refers to the content standards in the U.S. FDA Inactive Ingredients Database. | | | |

**Table 2**

| Composition | Content (w/v%) | Function |
|---|---|---|
| Sodium Salt Represented by Formula I | 5.0 | Active Material |
| Citric Acid | 0.01 | pH Buffer/Chelating Agent |
| Sodium Chloride | 0.35 | Inorganic tonicity agent |
| Sodium Thiosulfate Pentahydrate | 0.4 | Antioxidant |
| Benzalkonium Chloride | 0.02 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 3**

| Composition | Content (w/v%) | Function |
|---|---|---|
| Sodium Salt Represented by Formula I | 5.0 | Active Material |
| Sodium Phosphate, monobasic, Monohydrate | 0.5 | pH Buffer |
| Glycerol | 1.3 | Organic tonicity agent |
| Sodium Thiosulfate Pentahydrate | 0.3 | Antioxidant |
| Benzalkonium Chloride | 0.01 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 4**

| Composition | Content (w/v%) | Function |
|---|---|---|
| Sodium Salt Represented by Formula I | 3.0 | Active Material |
| Sodium Phosphate, monobasic, Monohydrate | 0.02 | pH Buffer |
| Propylene Glycol | 1.4 | Organic tonicity agent |
| Sodium Thiosulfate Pentahydrate | 0.4 | Antioxidant |
| Benzalkonium Chloride | 0.015 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 5**

| Composition | Content (w/v%) | Function |
|---|---|---|
| Sodium Salt Represented by Formula I | 5.0 | Active Material |
| Sodium Phosphate, monobasic, Monohydrate | 0.02 | pH Buffer |
| Propylene Glycol | 1.1 | Organic tonicity agent |
| Sodium Chlorite | 0.01 | Preservative |
| Water for Injection | Solvent | |
| pH (after the adjustment by 1 N NaOH or 1N HCl) | 7.2 ± 0.2 | |
| Osmolarity | 300 ± 20 mOsmol/L | |

**Table 6**

| Composition | Content (w/v%) | Function |
|---|---|---|
| Sodium Salt Represented by Formula I | 5.0 | Active Material |
| Sodium Phosphate, monobasic, Monohydrate | 0.02 | pH Buffer |
| Glycerol | 1.3 | Organic tonicity agent |
| Sodium Thiosulfate Pentahydrate | 0.3 | Antioxidant |
| Polyquaternium-1 | 0.001 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 7**

| Composition | Content (w/v%) | Function |
|---|---|---|
| Sodium Salt Represented by Formula I | 5.0 | Active Material |
| Sodium Phosphate, monobasic, Monohydrate | 0.02 | pH Buffer |
| Potassium Chloride | 0.1 | Inorganic tonicity agent |
| Sodium Chloride | 0.4 | |
| Sodium Thiosulfate Pentahydrate | 0.3 | Antioxidant |
| Polyquaternium-1 | 0.001 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 8**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | pH Buffer |
| Trehalose | 2.0 | Organic tonicity agent |
| Erythritol-NF | 2.0 | |
| Polyquaternium-1 | 0.005 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 9**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| L-Carnitine | 3.0 | Organic tonicity agent |
| Sodium Thiosulfate Pentahydrate | 0.3 | Antioxidant |
| Sodium Chlorite | 0.02 | Preservative |
| Water for Injection | Solvent | |
| pH (after the adjustment by 1 N NaOH or 1N HCl) | 7.2±0.2 | |
| Osmolarity | 300±20 mOsmol/L | |

**Table 10**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| Glycerol | 0.3 | Organic tonicity agent |
| Propylene Glycol | 0.1 | |
| Sodium Alginate | 0.50 | Comfort Agent |
| Sodium Chlorite | 0.01 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 11**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| Sodium Chloride | 0.28 | Inorganic tonicity agent |
| Sodium Hyaluronate (weight-average molecular weight of 800,000-1,200,000) | 0.15 | Comfort Agent |
| Sodium Chlorite | 0.005 | Preservative |
| Water for Injection | Solvent | |
| pH (after the adjustment by 1 N NaOH or 1N HCl) | 7.2±0.2 | |
| Osmolarity | 300±20 mOsmol/L | |

**Table 12**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| Sodium Chloride | 0.28 | Inorganic tonicity agent |
| Sodium Hyaluronate (weight-average molecular weight of 1,200,000) | 0.10 | Comfort Agent |
| Sodium Hyaluronate (weight-average molecular weight of 800,000) | 0.10 | |
| Sodium Chlorite | 0.005 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 13**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| Potassium Chloride | 0.4 | Inorganic tonicity agent |
| Carboxymethyl cellulose (weight-average molecular weight | 0.25 | Comfort Agent |
| of 400,000-600,000) | | |
| Sodium Chlorite | 0.005 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 14**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| Potassium Chloride | 0.4 | Inorganic tonicity agent |
| Carboxymethyl cellulose (weight-average molecular weight of 400,000-600,000) | 0.25 | Comfort Agent |
| Pluronic^{®}-F127 | 0.02 | Surfactant |
| Sodium Chlorite | 0.01 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 15**

| Composition | Content (w/v%) | Function |
|---|---|---|
| Sodium Salt Represented by Formula I | 5.0 | Active Material |
| Boric Acid | 0.35 | pH Buffer |
| Sodium Borate | 0.08 | |
| Potassium Chloride | 0.4 | Inorganic tonicity agent |
| Hyaluronic Acid | 0.2 | Comfort Agent |
| Carboxymethyl cellulose (weight-average molecular weight | 0.25 | Comfort Agent |
| of 400,000-600,000) | | |
| Pluronic^{®}-F127 | 0.02 | Surfactant |
| Sodium Chlorite | 0.01 | Preservative |
| Water for Injection | Solvent | |
| pH (after the adjustment by 1 N NaOH or 1N HCl) | 7.2 ± 0.2 | |
| Osmolarity | 300±20 mOsmol/L | |

**Table 16**

| Composition | Content (w/v%) | Function |
|---|---|---|
| Sodium Salt Represented by Formula I | 10.0 | Active Material |
| Sodium Phosphate, monobasic, Monohydrate | 0.02 | pH Buffer |
| Sodium Chloride | 0.1 | Inorganic tonicity agent |
| Sodium Thiosulfate Pentahydrate | 0.3 | Antioxidant |
| Benzalkonium Chloride | 0.02 | Preservative |
| Water for Injection | Solvent | |
| pH (after the adjustment by 1 N NaOH or 1N HCl) | 7.2 ± 0.2 | |
| Osmolarity | 300 ± 20 mOsmol/L | |

**Table 17**

| Composition | Content (w/v%) | IID Content Standards (w/v%) | Function |
|---|---|---|---|
| Free Acid of Sodium Salt Represented by Formula I | 5.0 | - | Active Material |
| NaOH | 0.5 | 0.4-1.85- | - |
| Sodium Phosphate, monobasic,Monohydrate | 0.02 | 0.041-1.3- | pH Buffer |
| Sodium Chloride | 0.4 | 0.65-0.9- | Inorganic tonicity agent |
| Sodium Thiosulfate Pentahydrate | 0.3 | 0.2-0.31- | Antioxidant |
| Benzalkonium Chloride | 0.01 | 0.005-1.0- | Preservative |
| Water for Injection | Solvent | | |
| pH (after the adjustment by 1 N NaOH or 1N HCl) | 7.2 ± 0.2 | | |
| Osmolarity | 300±20 mOsmo/L | | |

| | | | |
|---|---|---|---|
| Note: the "IID content standards" in Table 17 refers to the content standards in the U.S. FDA Inactive Ingredients Database. | | | |

### Stability test:

The VVN001-containing ophthalmic composition prepared in Example 1 was bottled in a multi-dose bottle (0.5 mL), a multi-dose bottle (1 mL), and a multi-dose bottle (2 mL), respectively, and stored at an ambient temperature of 40° C and a humidity of 75% RH. After 1 month and 3 months of storage, the stability test of the VVN001-containing ophthalmic composition in the above single-dose bottle (0.5 mL), multi-dose bottle (1 mL), and multi-dose bottle (2 mL) was carried out, and the results are shown in Table 18.

High-performance liquid chromatography (HPLC) was used to determine the single impurity content of VVN001-containing ophthalmic composition stored for different periods. Tables 19 to 20 show the single impurity content of VVN001-containing ophthalmic composition after 1 month and 3 months of storage, respectively.

**Table 18**

| Test Items | Specifica tions | single-d ose bottle (0.5 mL) | Multi-dos e bottle (1 mL) | Multi-dos e bottle (2 mL) | single-dos e bottle (0.5 mL) | Multi-dos e bottle (1 mL) | Multi-dos e bottle (2 mL) |
|---|---|---|---|---|---|---|---|
| Storage Period | - | 1 month | 1 month | 1 month | 3 months | 3 months | 3 months |
| Characteri stics | - | Clear Solution | Clear Solution | Clear Solution | Clear Solution | Clear Solution | Clear Solution |
| Sterility | Sterile | Sterile | Sterile | Sterile | Sterile | Sterile | Sterile |
| pH Value | 7.0 -7.4 | 7.01 | 7.13 | 7.20 | 7.11 | 7.11 | 7.07 |
| Osmolarity(mOsmo 1/L) | 280-320 | 320 | 313 | 313 | 317 | 301 | 314 |
| Total Impurity( %) | - | 1.68 | 1.70 | 1.72 | 1.31 | 1.23 | 1.34 |

| | | | | | | | |
|---|---|---|---|---|---|---|---|
| Note: The symbol "-" in Table 18 means the corresponding parameter is not available. | | | | | | | |

**Table 19**

| Single Impurity | single-dose bottle (0.5 mL) | | Multi-dose bottle (1 mL) | | Multi-dose bottle (2 mL) | |
|---|---|---|---|---|---|---|
| | Relative Retention Time | Peak Area (%) | Relative Retention Time | Peak Area (%) | Relative Retention Time | Peak Area (%) |
| 1 | 0.90 | 0.22 | 0.90 | 0.23 | 0.90 | 0.23 |
| 2 | 0.92 | 0.08 | 0.92 | 0.08 | 0.92 | 0.08 |
| 3 | 0.95 | 0.36 | 0.95 | 0.37 | 0.95 | 0.37 |
| 4 | 1.02 | 0.13 | 1.02 | 0.12 | 1.02 | 0.13 |
| 5 | 1.06 | 0.09 | 1.06 | 0.10 | 1.06 | 0.11 |
| 6 | 1.07 | 0.07 | 1.07 | 0.07 | 1.07 | 0.07 |
| 7 | 1.10 | 0.32 | 1.10 | 0.32 | 1.10 | 0.32 |
| 8 | 1.15 | 0.41 | 1.15 | 0.41 | 1.15 | 0.41 |
| Total Impurity Content | - | 1.68 | | 1.70 | - | 1.72 |

| | | | | | | |
|---|---|---|---|---|---|---|
| Note: The symbol "-" in Table 19 means the corresponding parameter is not available. | | | | | | |

**Table 20**

| Single Impurity | single-dose bottle (0.5 mL) | | Multi-dose bottle (1 mL) | | Multi-dose bottle (2 mL) | |
|---|---|---|---|---|---|---|
| | Relative Retention Time | Peak Area (%) | Relative Retention Time | Peak Area (%) | Relative Retention Time | Peak Area (%) |
| 1 | 0.89 | 0.05 | 0.89 | 0.04 | 0.89 | 0.04 |
| 2 | 0.92 | 0.09 | 0.92 | 0.06 | 0.92 | 0.13 |
| 3 | 0.95 | 0.07 | 0.95 | 0.06 | 0.95 | 0.05 |
| 4 | 0.95 | 0.20 | 0.95 | 0.20 | 0.95 | 0.17 |
| 5 | 1.02 | 0.11 | 1.02 | 0.12 | 1.02 | 0.15 |
| 6 | 1.06 | 0.09 | 1.06 | 0.09 | 1.06 | 0.10 |
| 7 | 1.10 | 0.28 | 1.10 | 0.26 | 1.10 | 0.29 |
| 8 | 1.14 | 0.42 | 1.15 | 0.41 | 1.14 | 0.41 |
| Total Impurity | - | 1.31 | - | 1.23 | - | 1.34 |

| | | | | | | |
|---|---|---|---|---|---|---|
| Note: The symbol "-" in Table 20 means the corresponding parameter is not available. | | | | | | |

From the stability data in Tables 19 to 20, it can be seen that the ophthalmic compositions prepared by the method of preparation of the present application, after being stored in the containers of single-dose bottle (0.5 mL), multi-dose bottle (1 mL), and multi-dose bottle (2 mL) for up to 3 months at an ambient temperature of 40°C and a humidity of 75% RH, have a good sterility, good stability, and extremely low impurity content. The relative retention times of the single impurity in Tables 19 and 20 is the ratio of the retention time of the single impurity to the retention time of the sodium salt represented by formula I.

### Clinical Trial:

The ophthalmic composition comprising 5 w/v% of VVN001 provided in present application was used in clinical trial. The subjects with moderately severe dry eye disease were administered by eye drops twice a day. The overall trend of improvement of dry eye symptoms in the subjects was highly significant, and multiple corneal areas, including total corneal tattooing, reached a level of statistical significance on day 84 (p < 0.05). The trend of increase in tear secretion in the subjects was significantly better than that of the blank solvent control. Blank solvent control means that the ophthalmic composition used in the subject does not contain VVN001.

The foregoing are only preferred examples of the present application and are not intended to limit the scope of protection of the present application. Any modifications, equivalent substitutions, improvements and the like made within the spirit and principles of the present application are included in the scope of protection of the present application.

The foregoing are only preferred examples of the present invention and are not intended to limit the present invention, and any modifications, equivalent substitutions, improvements and the like made within the spirit and principles of the present invention are included in the scope of protection of the present invention.

## Claims

1. A VVN001-containing ophthalmic composition comprising an active material and ophthalmic excipients, wherein
the active material is a sodium salt represented by formula I and/or a free acid thereof, and a content of the active material in the ophthalmic composition is 0.01 w/v% to 10 w/v%, preferably 0.1 w/v% to 7.5 w/v%, more preferably 1.0 w/v% to 5.0 w/v%;
the ophthalmic excipient comprises a pH buffering agent and a tonicity agent; a content of the pH buffering agent in the ophthalmic composition is 0.001 w/v% to 2.0 w/v%, preferably 0.005 w/v% to 1.0 w/v%, more preferably 0.005 w/v% to 0.5 w/v%; and
a pH of the ophthalmic composition is 6.5 to 7.8, preferably 7.0 to 7.4; and an osmolarity of the ophthalmic composition is 200 mOsmol/L to 400 mOsmol/L, preferably 250 mOsmol/L to 350 mOsmol/L, more preferably 280 mOsmol/L to 320 mOsmol/L,

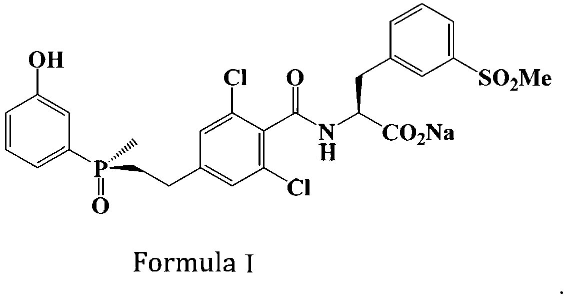

2. The ophthalmic composition according to claim 1, wherein the pH buffering agent is any one selected from the group consisting of boric acid, borate, citric acid, citrate, acetic acid-sodium acetate, trometamol hydrochloride, sodium bicarbonate, and phosphate;
the borate is at least one selected from the group consisting of sodium borate, potassium borate, and hydrates thereof;
the citrate is at least one selected from the group consisting of potassium citrate, sodium citrate, disodium hydrogen citrate, sodium dihydrogen citrate, dipotassium hydrogen citrate, potassium dihydrogen citrate, and hydrates thereof; and
the phosphate is one or more selected from the group consisting of disodium hydrogen phosphate, sodium dihydrogen phosphate, dipotassium hydrogen phosphate, potassium dihydrogen phosphate, and hydrates thereof.

3. The ophthalmic composition according to claim 2, wherein the pH buffering agent is phosphate; and a content of the phosphate in the ophthalmic composition is 0.001 w/v% to 2.0 w/v%, preferably 0.01 w/v% to 1.0 w/v%, more preferably 0.005 w/v% to 0.5 w/v%.

4. The ophthalmic composition according to claim 1, wherein the tonicity agent is an inorganic tonicity agent and/or an organic tonicity agent;
the inorganic tonicity agent is one or more selected from the group consisting of sodium chloride, potassium chloride, calcium chloride, zinc chloride and magnesium chloride; a content of the inorganic tonicity agent in the ophthalmic composition in an aqueous solution is 0.01 w/v% to 1.5 w/v%, preferably 0.2 w/v% to 1 w/v%; and
the organic tonicity agent is any one selected from the group consisting of erythritol, glucose, glycerol, propylene glycol, glycine, diglycine, alanine, taurine, tetrahydromethylpyrimidine carboxylic acid, mannitol, sorbitol, L-Carnitine, and trehalose; and a content of the organic tonicity agent in the ophthalmic composition is 0.001 w/v% to 10 w/v%, preferably 0.01 w/v% to 5 w/v%.

5. The ophthalmic composition according to claim 1, wherein the ophthalmic excipient further comprises a surfactant which is at least one selected from the group consisting of polyoxyethylene-polyoxypropylene-polyoxyethylene triblock copolymer, sodium dodecyl sulfate, polyethoxylated sorbitan fatty acid ester, polyoxyethylene alkyl ether, polyoxyethylene stearate, poloxamine, sorbitan fatty acid ester, polyethylene glycol, polyethoxylated fatty alcohol, polyoxyethylene 40 hydrogenated castor oil, docusate sodium, quaternary ammonium compounds, C₆-C₂₀ fatty acids, saccharide fatty acid ester, fatty acid glyceride, polysorbate, and tyloxapol; and a content of the surfactant in the ophthalmic composition is 0.001 w/v% to 1 w/v%.

6. The ophthalmic composition according to claim 1, wherein the ophthalmic excipient further comprises a comfort agent which is at least one selected from the group consisting of polyol, cellulose derivative, dextran, polyethylene glycol, polysorbate, povidone, trehalose, hyaluronic acid, carbomer, sodium hyaluronate and sodium alginate;
the polyol is at least one selected from the group consisting of glycerol, propylene glycol, polyvinyl alcohol and mannitol;
the cellulose derivative is at least one selected from the group consisting of hydroxypropylmethyl cellulose-E4M, hydroxypropylmethyl cellulose-LV, hydroxyethyl cellulose, hydroxymethyl cellulose, methylcellulose, hemicellulose and ethylcellulose; and
a content of the comfort agent in the ophthalmic composition is 0.001 w/v% to 2 w/v%.

7. The ophthalmic composition according to claim 1, wherein the ophthalmic excipient further comprises a chelating agent which is at least one selected from the group consisting of disodium ethylenediaminetetraacetate, alkali metal hexametaphosphate, nitrilotriacetic acid, ethylenediamine disuccinic acid, iminodisuccinic acid, methylglycine diacetic acid, L-glutamic acid N,N-diacetic acid, ethylenediamine-N,N'-diglutamic acid, ethylenediamine-N,N'-bismalonic acid, 3-hydroxy-2,2-iminodisuccinic acid, 2-hydroxyethyliminodiacetic acid, 2,6-pyridinedicarboxylic acid, diethylenetriaminepentaacetic acid, hydroxyethyldiaminetriacetic acid, 1,2-diaminocyclohexanetetraacetic acid, hydroxyethylaminodiacetic acid, polyphosphate, citric acid, citrate, tartaric acid, tartrate, and ethylenediaminetetraacetic acid; a content of the chelating agent in the ophthalmic composition in an aqueous solution is 0.001 w/v% to 1 w/v%; and
the citrate is at least one selected from the group consisting of potassium citrate, sodium citrate and hydrates thereof; and the tartrate is at least one selected from the group consisting of sodium tartrate, potassium tartrate, potassium hydrogen tartrate, sodium hydrogen tartrate and hydrates thereof.

8. The ophthalmic composition according to claim 1, wherein the ophthalmic excipient further comprises an antioxidant which is at least one selected from the group consisting of sodium thiosulfate, sodium metabisulfite, N-acetylcysteine, butylhydroxyanisole, and butylhydroxytoluene; and a content of the antioxidant in the ophthalmic composition is 0.001 w/v% to 1 w/v%.

9. The ophthalmic composition according to claim 1, wherein the ophthalmic excipient further comprises a preservative which is at least one selected from the group consisting of benzalkonium chloride, sodium chlorite, polyquaternium-1, sorbic acid, ethylenediaminetetraacetic acid, boric acid, sodium borate, sodium bisulfate, sodium thiosulphate, ascorbate, urea peroxide, and benzalkonium bromide; and a content of the preservative in the ophthalmic composition is 0.001 w/v% to 0.1 w/v%.

10. Use of the ophthalmic composition according to any one of claims 1 to 9 in the preparation of a medicament for treating dry eye disease.
